(19) Europäisches Patentamt
European Patent Office
Office européen des brevets

(11) **EP 1 214 579 B1**

(12) **EUROPEAN PATENT SPECIFICATION**

(45) Date of publication and mention
of the grant of the patent:
**18.10.2006 Bulletin 2006/42**

(51) Int Cl.:
***G01N 21/35*** (2006.01)

(21) Application number: **00955997.2**

(22) Date of filing: **31.08.2000**

(86) International application number:
**PCT/CA2000/001005**

(87) International publication number:
**WO 2001/016579 (08.03.2001 Gazette 2001/10)**

(54) **METHOD OF CALIBRATING A SPECTROSCOPIC DEVICE**

VERFAHREN ZUM KALIBRIEREN EINER SPEKTROSKOPIEVORRICHTUNG

PROCEDE D'ETALONNAGE D'UN DISPOSITIF SPECTROSCOPIQUE

(84) Designated Contracting States:
**AT BE CH CY DE DK ES FI FR GB GR IE IT LI LU MC NL PT SE**

(30) Priority: **31.08.1999 US 151536 P**

(43) Date of publication of application:
**19.06.2002 Bulletin 2002/25**

(73) Proprietor: **NIR Diagnostics Inc.**
**Campbellville ON L0P 1B0 (CA)**

(72) Inventor: **CADELL, Theodore, E.**
**Conestogo, Ontario N0B 1N0 (CA)**

(74) Representative: **Warren, Anthony Robert et al**
**BARON & WARREN,**
**19 South End,**
**Kensington**
**London W8 5BU (GB)**

(56) References cited:
WO-A-99/34193          US-A- 5 362 965
US-A- 5 576 544         US-A- 5 606 164
US-A- 5 680 320

• SMALL G W ET AL: "STRATEGIES FOR COUPLING DIGITAL FILTERING WITH PARTIAL LEAST-SQUARES REGRESSION: APPLICATION TO THE DETERMINATION OF GLUCOSE IN PLASMA BY FOURIER TRANSFORM NEAR-INFRARED SPECTROSCOPY" ANALYTICAL CHEMISTRY,US,AMERICAN CHEMICAL SOCIETY. COLUMBUS, vol. 65, no. 22, 15 November 1993 (1993-11-15), pages 3279-3289, XP000503258 ISSN: 0003-2700

## Description

The present invention relates to the fields of spectroscopy, spectrophotometry, and chemometrics. In particular, the present invention relates to a method of calibrating a spectroscopic device for predicting analyte levels in a non-invasive manner. The method of the present invention is particularly suitable for blood glucose prediction based on near-infrared spectrophotometry measurements.

Biotechnological analysis and examination are often based on the measurement of various chemical analytes in the composition of a biological matrix such as blood, interstitial fluid, or living tissue. Such measurements may be used to evaluate a patient's state of health and to determine what, if any, treatment is necessary. For example, the frequent monitoring of blood glucose levels in diabetic persons with glucose meters is often necessary to allow such persons to manage the diabetes mellitus disease, by taking insulin injections or oral drugs to lower blood glucose when required. Intensive treatment based on frequent blood glucose measurements can significantly reduce the incidence of blindness, kidney loss, and other complications associated with diabetes.

Most home-based glucose measurement systems require the patient to invasively collect a blood sample, by pricking his or her finger, placing the sample on an appropriate test strip, and then testing the sample in an optical glucose meter. For millions of diabetics around the world, the use of lancets or other sharp instruments to draw blood for monitoring their insulin levels is a painful process, and one that often builds up calluses on fingers, making the collection of blood even more difficult. This invasive procedure may be especially difficult to perform on children and therefore particularly trying on parents. Furthermore, the test strips required for each blood sample are generally not reusable, and when multiple measurements are taken each day, amount to significant costs from the patient's point of view. Thus, despite the fact that a large number of diabetics should take several measurements throughout each day (for some individuals, physicians recommend testing glucose levels from 4 to 7 times daily), due to the pain, cost, and inconvenience involved, many diabetics do not monitor their glucose levels frequently enough.

Spectroscopy is based on the analysis of how incident radiation interacts with the vibrational and rotational states of molecules which are of analytical interest. Spectroscopic measurement techniques have gained increased popularity because of the ability to provide fast and non-invasive measurements of concentrations of different chemicals or analytes. For the reasons indicated above, this is particularly desirable for home based glucose meters. Spectrophotometry is a type of spectroscopy commonly used to quantitatively measure concentrations of analytes based on spectral energy distribution in the absorption spectrum of a sample solution or medium. In spectrophotometry, the energy distribution is typically analyzed within a range of the visible, ultraviolet, infrared, or near-infrared spectra. For example, near-infrared radiation (NIR) is electromagnetic radiation having a wavelength of between about 0.75 and 2.5 micrometers (i.e. from 150 to 400 THz). Near-infrared spectrophotometry generally uses instruments with quartz prisms in monochromators and with lead sulfide photoconductor cells as detectors to observe absorption bands, and NIR spectrophotometry is increasingly being used to measure in vivo analytes such as glucose, total hemoglobin, albumin, cholesterol, and ethanol.

Non-invasive, spectrophotometric measurement of glucose in human beings is performed by focusing an incident radiation source (or sources) on a specific part of the body and detecting the spectral distribution of the radiation transmitted therethrough. The absorbance of light from the incident radiation is due to the chemical components within that body part including water, fat, protein, hemoglobin, melanin, and glucose. One difficulty with glucose measurement spectral analysis, is the spectral overlap between glucose and other chemicals found in blood, often in much greater quantities than glucose. In addition, the thickness, color, and structure of the skin, bones, and blood through which the incident radiation passes will affect the transmission (or reflection/absorption) thereof. Furthermore, the concentration of analytes can vary with changes in activity level, diet, hormone fluctuations, and other factors. Glucose concentration measurements are also particularly susceptible to variations in physical and chemical conditions including temperature, pressure, humidity, and skin hydration. As a result, to perform a reliable non-invasive glucose prediction, NIR spectral measurements should be performed through a vascular equilibrated region of the body, and a NIR glucose spectrophotometer must be carefully designed so that the quality of raw spectral information from an NIR glucose meter is high. See generally Waynant and Chenault, "Overview of Non-Invasive Optical Glucose Monitoring Techniques", IEEE LEOS Newsletter, vol. 12, no. 2 (April 1998); and Burmeister and Arnold, "Spectroscopic Considerations for Noninvasive Blood Glucose Measurements with Near Infrared Spectroscopy", IEEE LEOS Newsletter, vol. 12, no. 2 (April 1998).

Near-infrared glucose measurements are generally suitable for tissue depths ranging from about 1 mm to 10 cm, and are often performed through a patient's finger tip, although other areas of the body (for example the web between two fingers, an ear lobe, or the upper lip) can also be used. The sample thickness is an important experimental parameter because a greater thickness increases the amount of absorption and thereby lowers the minimum limits for detection, whereas because less incident radiation successfully traverses through a thicker sample (i.e. without being absorbed) effectively increasing the spectral noise: see Burmeister and Arnold, supra.

In addition to the difficulties of obtaining accurate spectral measurements with NIR spectrophotometry, a more significant difficulty associated with such measurements has been the need to calibrate such an instrument so that it may be used by various different individuals, whose analyte concentrations and variability, for example, may differ substantially. Calibration of spectrophotometers (and of analytical instruments in general) is necessary to ensure the accuracy of measurements performed by such devices.

Two approaches to calibrate and predict analyte (e.g. glucose) levels have been used in the prior art. In the first approach, a thorough calibration process is performed separately on each individual with whom the instrument is to be used. This individual calibration process requires taking a number of blood samples over a period of time from the individual, and obtaining reference glucose concentration measurements from these samples. A number of corresponding non-invasive spectroscopic measurements are taken concurrently, and calibration regression analysis is then performed to correlate, e.g. through linear regression analysis, the non-invasive measurements with the reference measurements. In this manner, the spectroscopic device or spectrophotometer is custom calibrated to the specific individual. A major disadvantage of this approach, however, is that the calibration model is limited to data from that particular individual which may have an insufficient amount of information regarding variation of spectra overlapping (interfering with) those of the analyte of interest. It is important to be able to characterize such spectra to enable highly accurate analyte predictions. To alleviate this problem, the custom calibration process may have to be carried out frequently, even on a daily basis.

An alternative calibration approach requires that a universal or general calibration algorithm be developed. This approach is based on the notion that by making a large number of calibration measurements (as in the first approach) a single calibration model that sufficiently accounts for all individual variability of all overlapping or interfering analytes can be calculated. Chemometrics, the application of mathematical, statistical and formal logic methods to chemistry, is generally used to process and compute the spectral intensity data and to produce a calibration model: see Small and Arnold, "Data Handling Issues for Near-Infrared Glucose Measurements", IEEE LEOS Newsletter, vol. 12, no. 2 (April 1998); and Shaffer, Small, and Arnold, "Genetic Algorithm-Based Protocol for Coupling Digital Filtering and Partial Least-Squares Regression: Application to the Near-Infrared Analysis of Glucose in Biological Matrices," Anal. Chem. 68, 2663-2675 (1996). However, for measurements of glucose, and other analytes which vary distinctly and differently from one individual sample to another, a single universal calibration algorithm is often ineffective and can result in significant, and sometimes dangerous, erroneous predictions of analyte concentration levels.

Protocols are also established for coupling digital filtering techniques with partial least-squares (PLS) regression for use in constructing multivariate calibration models from Fourier transform near-infrared absorbance spectra. In Small et al., "Strategies for Coupling Digital Filtering with Partial Least-Squares Regression: Application to the Determination of Glucose in Plasma by Fourier Transform Near-Infrared Specroscopy", Analytical Chemistry, vol. 65 (1993) 3279 -3289, Gaussian-shaped bandpass digital filters were implemented by use of Fourier filtering techniques and employed to preprocess spectra to remove variation due to the background absorbance of the plasma matrix.

US 5,680,320 describes a method for quantifying the amounts of chemicals such as wet strength agents, starches and retention agents, hydrophobic agents and debonding agents, reacted or retained with the cellulose fibers in fluff pulp or paper. The method develops a calibration model by registering absorption, reflectance or emission spectral raw data of reference samples of paper or pulp containing known amounts of the performance chemicals to develop a learning set; processing the spectral raw data from the registered spectra to reduce noise and adjust for drift and diffuse light scatter; performing a data analysis on the learning set in which the processed spectral data of the reference samples are transferred into latent variables based on principal component analysis, and applying chemometric techniques on the latent variables in order to find the mathematical expression of the calibration model; and registering absorption, reflectance or emission spectral raw data of a sample of fluff paper or pulp containing unknown amounts of chemicals, processing the spectral raw data; transferring the processed spectral data into latent variables, and applying the developed calibration model on the latent variables in order to determine the unknown amounts of the reacted or retained performance chemicals.

US 5,606,164 describes method and apparatus for measuring the concentration of an analyte present in a biological fluid. The method includes the steps of applying NIR radiation to calibration samples to produce calibration data, analyzing the calibration data to identify and remove outliers, constructing a calibration model, collecting and analyzing unknown samples to identify and remove outliers, and predicting analyte concentration of non-outliers from the calibration model. Analysis of the calibration data includes data pretreatment, data decomposition to remove redundant data, and identification and removal of outliers using generalized distances. The calibration model may utilize principal component regression, partial least squares, multiple linear regression, or artificial neural networks, and reduction using principal component analysis or partial least squares scores. Unknown sample data is analyzed using data pretreatment followed by projection into the calibration model space, and identification and removal of outliers, with prediction of analyte concentration using the calibration model.

US 5,362,965 describes a method for determining oxygenate content and/or octane of hydrocarbon fuels suitable for automotive vehicles.

US 5,576,544 describes a method for providing general calibration of near-infrared quantitative analysis instruments. The method compares an individual's near-infrared spectrum to a plurality of near-infrared spectral clusters. Each near-infrared spectral cluster has a set of calibration constants. The calibration constants of the spectral cluster most closely associated with the individual spectra are used to custom calibrate the near-infrared analysis measurement instrument.

Thus, while NIR measurements provide a non-invasive, fast, painless, and convenient technique to monitor glucose levels, correlation and clinical interpretation of spectral measurements to obtain the true glucose levels is crucial for proper therapy and disease management. Proper calibration of instruments for different patient populations (which will vary in ethnicity, age, weight, and so on) is crucial in obtaining accurate glucose prediction models. Furthermore, careful validation and testing of the non-invasive results and the glucose prediction equation is needed to determine if the glucose correlation is consistent in all clinically important conditions and for all, or at least most, types of patients.

The present invention is directed to a method of calibrating a spectroscopic device for providing a non-invasive measurement of an analyte level in a test sample, generally as disclosed in the said G.W. Small et al., Analytical Chemistry, vol. 65 (1993) 3279-3289 document and in the preamble of claim 1, comprising:

(a) providing a plurality of calibration algorithms;

(b) taking a set of non-invasive spectral measurements using the spectroscopic device and corresponding reference measurements of the analyte concentration for each of a plurality of samples;

(c) using each of the calibration algorithms to calculate a set of predicted analyte concentration levels for the non-invasive spectral measurements taken in step (b);

(d) for each set of predicted analyte concentration levels obtained in step (c) determining the following:

a range of the analyte concentration level variability;

a slope of predicted analyte concentration versus corresponding reference analyte concentration;

$R^2$ wherein R is the correlation of the predicted analyte concentration with the reference analyte concentration; and

a standard error of prediction (SEP) wherein SEP is the square root of the mean of the squared deviations of the reference analyte concentrations from the predicted analyte concentrations; and

(e) selecting a calibration algorithm suitable for calibrating the spectroscopic device.

According to this aspect of the present invention, as defined in the characterizing clause of claim 1, the selection step comprises:

(i) selecting the sets of predicted analyte concentration levels in which the SEP is less than an upper error limit, the variability range is greater than a lower range limit, and the slope is between a lower slope limit and an upper slope limit, the lower and upper slope limits defining an acceptable slope range;

(ii) for each of the predicted sets selected in step (i), calculating a suitability score in response to the slope, the $R^2$ and the SEP for that predicted set, and selecting the calibration algorithm corresponding to the predicted set having the optimal, i.e. the highest, suitability score as the suitable calibration algorithm;

(iii) if no predicted sets are selected in step (i), selecting the sets of predicted analyte concentration levels in which the variability range is lower than the lower range limit and in which the SEP is less than the upper error limit;

(iv) from the predicted sets selected in step (iii), selecting the calibration algorithm corresponding to the predicted set having lowest SEP as the suitable calibration algorithm; and

(v) if no predicted sets are selected in step (i) and (iii), determining that no calibration algorithm is suitable.

Preferably, the acceptable slope range is subdivided into a plurality of subranges corresponding to a plurality of levels, comprising a first level and one or more subsequent levels, and if no predicted sets are selected in the first level, the method then includes repeating step (i) at each subsequent level until a predicted set is selected or there are no more subsequent levels.

The lower slope limit may be less than one and the upper slope limit may be greater than one. More specifically, the lower slope limit may be about 0.3 and the upper slope limit may be about 1.5. Alternatively, the acceptable slope range may be defined by a lower slope limit of about 0.3 and an upper slope limit of about 1.05.

Preferably, the plurality of calibration algorithms in step (a) are generated by the steps of:

(i) compiling data sets for each of a plurality of individuals by taking non-invasive spectral measurements using the spectroscopic device and corresponding reference measurements of analyte concentration for each of a number of samples from each individual;

(ii) rejecting data sets that are not suitable for calibration, wherein the data sets not suitable for calibration comprise:

data sets that contain at least some non-invasive spectral measurements that saturate the spectroscopic device;

data sets with a small range of variability of the non-invasive spectral measurements; and/or

data sets that are correlated to another variable;

(iii) combining data sets that are suitable for calibration into a plurality of groups depending on whether correlations of the combined data sets meet predetermined criteria; and

(iv) generating a calibration algorithm for each of the groups of data sets.

The predetermined criteria in step (iii) may include minimizing correlations of the analyte level in the combined data sets in a particular group with other parameters and maximizing the correlation between data sets in a particular group.

Steps (iii) and (iv) may be performed using partial least-squares regression analysis.

Steps (a), (c), and (d) may be performed on a computer associated with the spectroscopic device. Furthermore, the test sample may be an individual patient, the spectroscopic device may be a near-infrared spectrophotometer, and the analyte may be selected from the group consisting of glucose, hemoglobin, albumin, cholesterol, and ethanol.

Reference will now be made to the accompanying drawings which illustrate, by way of example, a preferred embodiment of the invention, and in which:

Figure 1 is a correlation scatterplot of glucose predictions for a number of different patients based on a universal, single calibration model;
Figure 2 is a correlation scatterplot of glucose predictions for a number of patients based on the multiple algorithm calibration model embodying the present invention;
Figure 3 is another correlation scatterplot of glucose predictions for a number of patients based on the multiple algorithm calibration model embodying the present invention;
Figure 4 is a correlation scatterplot of glucose predictions for a typical patient based on a universal, single calibration model; and
Figure 5 is a correlation scatterplot of glucose predictions for the same typical patient based on the multiple algorithm calibration model embodying the present invention.

As mentioned, the present invention relates to a method of calibrating a spectroscopic device for predicting analyte levels in a noninvasive manner. The present invention can be used with a typical NIR spectrophotometer system having a light source which is projected through the item to be examined, a sample interface mechanism, a spectrometer to separate the light into its component wavelengths, a detector, amplification electronics and a computer. By measuring the loss (absorption), between the source and the detector and applying appropriate chemometric (mathematical) techniques, it is possible to non-invasively determine the chemicals being examined since different chemicals absorb different amounts of light at different wavelengths. Such a spectrophotometric device and method are described in detail in United States Patent No. 5,361,758. While the present description relates primarily to glucose measurement, one of the major fields of application for NIR measurement at present, it will be understood that the principles of the present invention equivalently apply to other analytes non-invasively measured using various spectroscopic techniques.

To utilize the NIR spectrum for glucose measurement, it is necessary to use a spectrometer which has wide dynamic range, a high signal to noise ratio, and exhibits low scattering losses. The output from the spectrometer is used to generate spectra with high precision both in absorbance and wavelength. Significant glucose absorption bands are centered about wavelengths of 1.67, 2.13, 2.27, and 2.33 micrometers (as discussed in Small and Arnold, "Data handling Issues for Near-Infrared Glucose Measurements", supra). In addition to these wavelengths there are significant glucose absorption bands at 0.97 and 1.12 micrometers which wavelengths allow transmission measurement through a greater distance in the tissue. To be able to use NIR to measure a particular compound/analyte, chemometric mathematical analysis is applied to the measured spectrum. The mathematical analysis techniques are carried out by a computer equipped with advanced software capable of interpreting the resulting complex spectra.

To universally calibrate an NIR spectrophotometer, the absorbance associated with the compound or analyte of interest must first be measured on a relatively large number of samples. These NIR measurements are then compared to measurements made in a more traditional and more accurate invasive manner. From these comparisons an algorithm is developed that characterizes the analyte to be measured. The methods used to generate calibration models for NIR spectrophotometers are sophisticated. An important criterion is that calibration samples be uncorrelated with other chemical species concentrations. Satisfaction of these criteria is burdensome because of the large number of chemical species typically present in a biological matrix or growth medium.

The present invention provides a multi-algorithm calibration method for use with a spectroscopic device capable of predicting analyte levels in a biological matrix, such as blood glucose levels, for a variety of different samples with significantly greater accuracy than prior art universal single calibration models. The calibration method of the invention has two key parts: the generation of a plurality of different algorithms and the selection of the algorithm which is most appropriate for generating an accurate prediction of analyte concentration.

To generate suitable calibration algorithms, the non-invasive and reference measurement data sets for a number of individuals are first compiled. Once this is achieved, correlation techniques are carried out to relate non-invasive spectral measurements to reference glucose levels. At this stage the suitability of individual data samples for calibration purposes must be considered. If some of the non-invasive spectral measurements in a data set result in a saturation of the measurement equipment, e.g. at the output of an analog-to-digital converter, then the data sample should be disqualified for use in a calibration algorithm. Similarly, if the range of variability of the non-invasive spectral measurements in a data set is too small, the data sample should also be disqualified from calibration. Furthermore, if the correlation of glucose in a particular data set is undesirably correlated to another variable, such as the concentration of another chemical, the sample should again be disqualified. As a result, a large numbers of sample sets will not be suitable for calibration algorithm development.

The grouping or combining of calibration-suitable data sets (each specific to a particular individual) into different algorithms occurs on a trial and error basis, by repeatedly combining these data sets and performing a new correlation on the combined sets of data. If the correlations of the combined sets meet certain criteria, then the combination is acceptable, if not, a different combination is attempted. Once a combination is deemed acceptable, the addition of a further data set is attempted and its acceptability is determined in turn. In this manner, a combination of data sets for an algorithm grows from 2 data sets, to 3 data sets, to 4 data sets, and so on until a sufficiently large number of data sets is obtained from which a reliable calibration algorithm can be developed.

In combining data sets for a single algorithm, two criteria are key. First, any correlation of glucose in the combined data set group with other parameters must be minimized. Such undesirable correlations may be exhibited with different chemical species concentrations or other time varying quantities. A suitable quantitative test for this criteria is to ensure that all undesirable correlations of the combined data sets, as indicated by the square of the correlation coefficient $R_u^2$ (for the undesirable correlations), are less than a certain limit, such as $R_u^2 < 0.10$. In known manner, the correlation coefficient, $R_u$, is a measure of the tendency of two variables to vary together. If the addition of a data set to another data set or to a suitable sub-combination of data sets does not satisfy this criteria, the new combination will not be acceptable.

In addition, the combination of data sets can be optimized by ensuring that the data sets exhibit a high degree of correlation with one another, so a second criteria in combining data sets should require that the data sets in a particular group be sufficiently correlated with one another, This can be achieved, for example, by ensuring that the Standard Error of Prediction (SEP) is minimal and the square of the correlation coefficient, $R^2$, together with the slope relating reference glucose to predicted glucose, are greater than about 0.8 for an existing sub-combination of data sets and another data set being potentially combined with that sub-combination.

In an alternate embodiment of the present invention, instead of successively adding on to, or building up, groups of data sets for a calibration algorithm, a large group of calibration suitable data sets can be initially taken up at random. In this embodiment, data sets are removed from the group, and the SEP and correlation criteria above are reevaluated. If the SEP, slope and correlation criteria improve after the removal of the data set, the data set is permanently removed from the group, whereas if the criteria do not improve, the data set is reinstated back into the group. Using this top-down approach, the removal of data sets can cease when the criteria meet acceptable limits.

The SEP, slope, and correlation calculations for determination of the calibration algorithm groupings (and the associated calibration algorithms) become increasingly complex with increasing amounts of data and are preferably carried out using partial least-squares (PLS) regression analysis. The PLS analysis techniques are well known to those skilled in the art and provide good linear approximation by removing considerable redundant information: see generally Small and Arnold, "Data handling Issues for Near-Infrared Glucose Measurements", supra. Other types of analysis, such as partial component analysis (PCA) or artificial neural networks (ANN), can also be used.

This calibration algorithm generation process is used to generate at least two groups of data sets (corresponding to groups of individuals), each providing a calibration model or algorithm for predicting the level of analyte present in a biological matrix based on a non-invasive spectroscopic measurement. Preferably, two initial groups of data set are developed, and, from these two groups, additional groups can be generated. These additional groups are generally smaller in size than the two initial groups and may meet more stringent correlation criteria. These additional groups may be constructed with the top-down approach described above, wherein data sets are selectively removed from an initial group to determine if the correlation criteria improve or deteriorate after the removal.

Given the availability of multiple calibration algorithms, it is necessary to select the most suitable of these algorithms for predicting the levels of analyte in any given patient or sample. Once this is done the spectroscopic device is calibrated with the selected algorithm. The algorithm selection process is generally independent of the process used to generate the multiple algorithms, although it will clearly depend on the number of different algorithms that are available.

For any given patient or sample on which the non-invasive spectroscopic measurement is to be made, it is necessary first to compile a set of highly accurate reference measurements and corresponding non-invasive spectral measurements at selected intervals over a period of time. In a preferred embodiment, two successive non-invasive spectral measurements are successively taken each measurement interval, and their mean is then taken as a single non-invasive measurement for that interval.

Before algorithm selection begins, two variables may be set to determine the stringency of acceptance requirements for patients (i.e. whether at least one of the available calibration algorithms will be found suitable for a patient). These are (i) a lower range limit for the range of glucose or analyte variability in the data set (if the data does not vary significantly it may not provide sufficient information to be useful), and (ii) an upper error limit ($SEP_{max}$) for the standard error of prediction (SEP), i.e. the square root of the mean of the squared deviations of the reference analyte concentrations from the analyte concentration values predicted by a calibration algorithm.

From the non-invasive spectral measurements, a predicted set of analyte concentration levels are calculated according to each calibration algorithm. When compared to the reference set of concentration levels, each predicted set will be calculated from a linear regression or "best fit" line characterized by: a range of glucose level variability; a slope (which ideally equals 1, i.e. the predicted values correspond directly to the reference values); a correlation coefficient R, defining the correlation of the predicted value set with the reference value set; and a standard error of prediction (SEP), as defined above. Note that as the correlation coefficient R increases the SEP will decrease, and vice versa, except in cases in which there is an offset or bias in the predicted analyte.

By way of example, and not to restrict the scope of the present invention, the algorithm selection process can include 4 levels or steps. In a first level, if the predicted data set for an algorithm meets the following criteria

    i) SEP < upper error limit ($SEP_{max}$)
    ii) Analyte range > lower range limit
    and iii) 0.5 < slope (of regression line) < 1.05,

a suitability score is calculated according to the following equation:

$$score = (slope)\,(R^2)\,/\,(SEP)$$

If more than one algorithm meets the first level criteria, the algorithm with the highest suitability score is selected and the selection process is complete. If no algorithm qualifies in terms of the first level criteria, the selection process advances to a second level.

The second level is identical to the first level, except that the slope criteria becomes

$$0.33 < slope\ (of\ regression\ line) < 0.5$$

For algorithms that meet this revised slope criterion (and the SEP and range criteria), a suitability score is calculated as in the first level. Again, if more than one algorithm qualifies at the second level, the algorithm with the highest score is selected, and the selection process is complete.

At a third level, if none of the algorithms qualify at either the first level or the second level and the range of analyte measurements is greater than the lower range limit, the sample or patient is excluded from measurement, since under these circumstances no calibration algorithm can be relied on to perform accurately.

Lastly, at a fourth level, if none of the algorithms qualify at either the first level or the second level and the range of analyte measurements is lower than the lower range limit, the algorithm with the lowest SEP is selected if that SEP is less than the upper error limit. If the SEP is greater than the upper error limit, the patient is again excluded from measurement. Thus, in the fourth level, the SEP becomes the sole factor in deciding whether an algorithm should be accepted.

Note that the slope criteria limits in the first and second levels described above are preferred limits, and these levels can also be varied somewhat (similar to the lower range limit and upper error limit) depending on the application and acceptance requirements. Also, the number of levels or steps used in the algorithm selection process can vary, from a single level covering the entire preferred range, to two or more levels subdividing the preferred range into two or more range limits or range criterion. Regardless of the number of levels, however, the third and fourth levels as described above will correspond to the last two levels, in which the patient is either excluded, or a decision to accept an algorithm is made on the basis of SEP alone. It will be readily appreciated by those skilled in the art that the number of levels or steps chosen is a matter of preference only.

To illustrate the principals of the present invention, the multiple algorithm calibration method was used to calibrate a NIR spectrophotometer for measuring glucose levels in a number of different patients. All reference data measurements were taken with a glucose analyzer such as the industry standard YSI™ (Yellow Springs Instruments) Glucose Analyzer which provides precise glucose measurements on (invasive) blood samples. The inventor found that only approximately 6% of single NIR patient data sets qualified for calibration purposes, given the criteria described above. These calibration-qualified sets of data were grouped into two initial calibration algorithm sets, each initial group was built up to a group with 33 sets of data within it. Eleven additional groups were spun off from these two initial groups and each of these groups had at least 8 sets of data within it. Thus, thirteen different calibration algorithms were available for predicting glucose levels.

The algorithm selection was based on a total of 24 reference measurements (i.e. YSI™ measured) and 24 means of two NIR finger measurements, taken from a patient over a three day period. The above described algorithm selection process was run for a number of different patients (not involved in the calibration process).

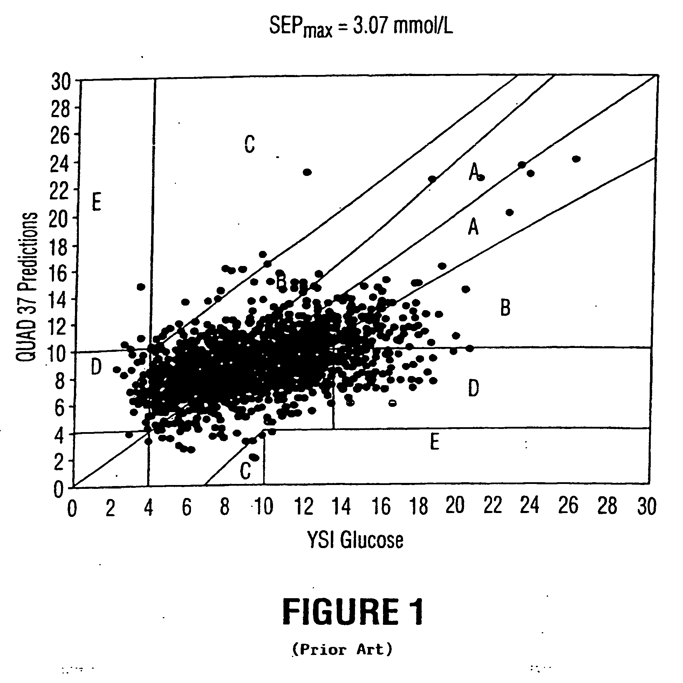

Figure 1 shows a correlation scatterplot of glucose predictions for a number of different patients based on a universal calibration model (QUAD 37). The scatterplot in Figure 1 is superimposed on a Clarke error grid, as described in Clarke and Cox, "Error Grid Analysis", Diabetes Care, 10:622-628 (1987). The Clarke error grid breaks the correlation space into five regions (A-E) that assess measurement accuracy on the basis of validity of the corresponding clinical decision. Correlation points falling within the "A" region correspond to the correct clinical decision being made based on the similarity between the actual and predicted glucose levels. (i.e. the predicted values deviate by no more than 20% from the reference values). In the "B" region, the predicted values deviate by more than 20% from the reference values, but treatment decisions made based on the predicted levels of glucose would not jeopardize or adversely affect the patient. In regions "C", "D", and "E", the predictions significantly deviate from the reference values, and treatment decisions based on these predictions may well be harmful to a patient. The Clarke error grid is often used to evaluate the clinical consequences of home-use blood glucose monitor errors, in contrast to more conventional analytical methods which may be more suitable for laboratory reference devices.

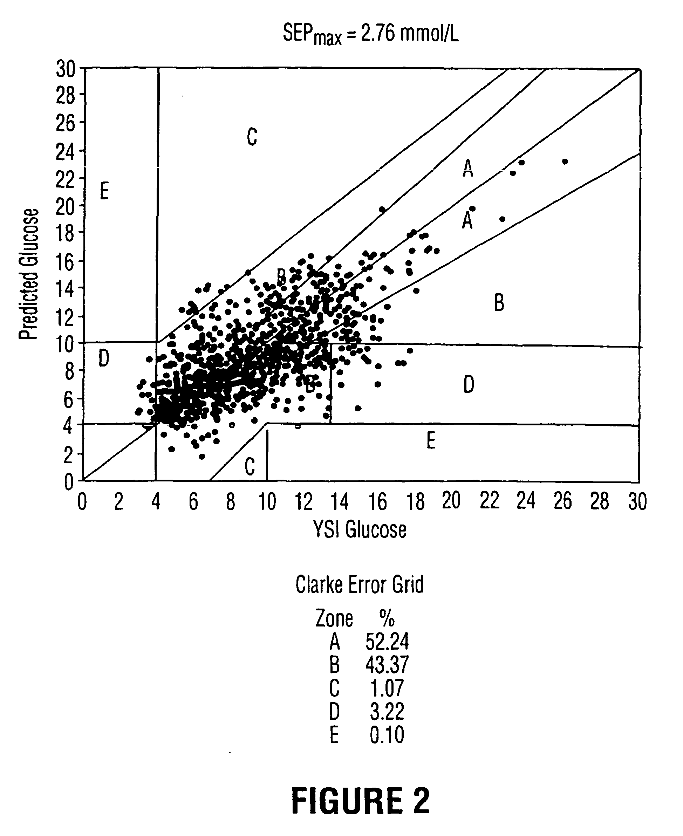

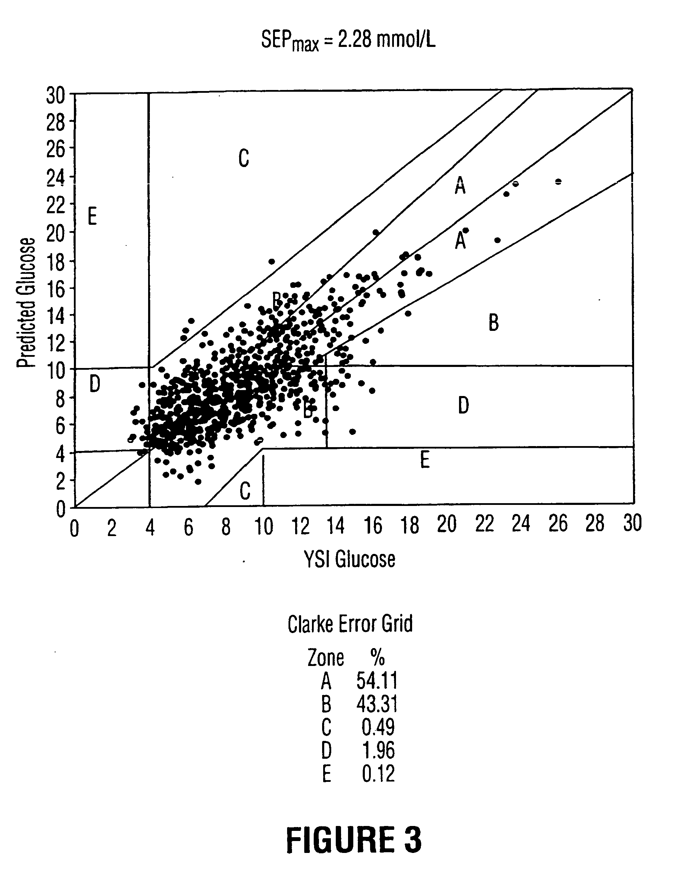

Referring to Figure 1, it can be seen that the upper error limit $SEP_{max}$ for this universal calibration algorithm is 3.07 mmol/L and that significant amounts of the data are outside acceptable limits or regions. In contrast, Figure 2 shows the correlation scatterplot of glucose predictions for 25 patients based on the multiple algorithm calibration model according to the present invention, with $SEP_{max}$ equal to 2.76 mmol/L. Similarly, Figure 3 shows a correlation scatterplot of glucose predictions for 22 patients based on the multiple algorithm calibration model according to the present invention, with

$SEP_{max}$ equal to 2.28 mmol/L. Both the predictions in Figure 2 and particularly Figure 3 exhibit much better accuracy than those in Figure 1 (calibrated according to a universal algorithm).

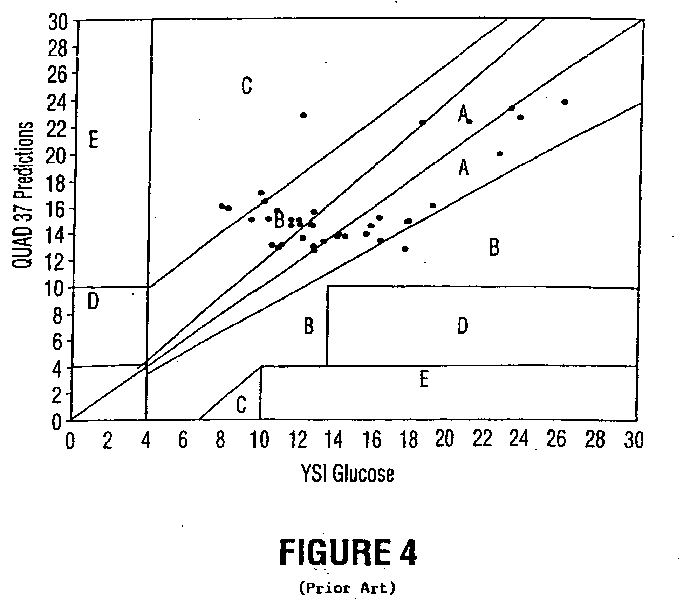

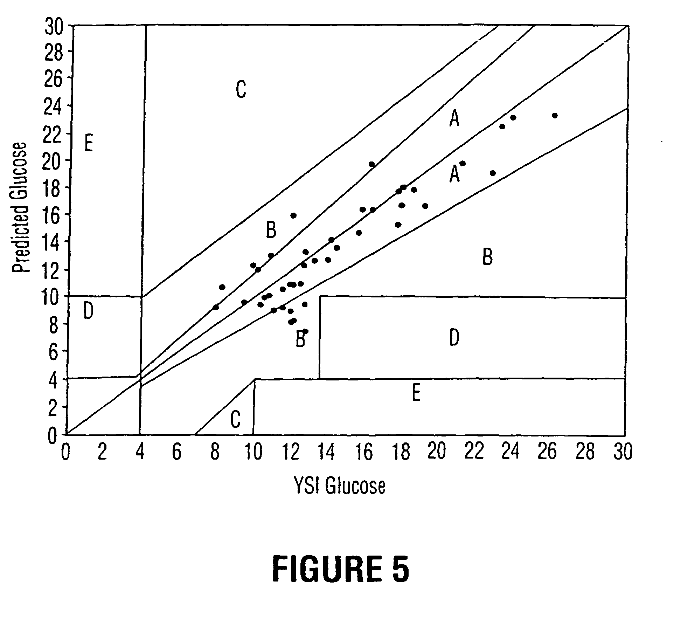

Similarly, Figure 4 shows a set of correlation data for a typical patient, calibrated according to the same universal algorithm, whereas Figure 5 shows the correlation data for the identical patient calibrated according to the multiple algorithm method of the present invention. Once more, the improved accuracy of the glucose predictions based on the calibration method of the present invention is evident.

It will be clear to those skilled in the art that the principles of the present invention are applicable to a broad range of spectroscopic applications. For example, NIR radiation can be used to penetrate such items as human tissue, vials of blood, or containers of milk - all normally considered opaque. Mathematical analysis of the resulting absorption spectrum determines the composition of the substance penetrated by the light. Thus, in a blood testing lab, samples can be examined without contact by passing the light through a plastic vial containing the liquid. Similarly, in a dairy application, the NIR light is passed through a bag of milk and such parameters as butterfat, solid particles and lactate are measured. The multiple algorithm method of the present invention is easily extendible to these types of non-invasive measurements as well. Furthermore, the method of the present invention can also be applied to the non invasive measurement of a number of other blood analytes such as cholesterol, hemoglobin, Hb1Ac, Fructosamine, and 1.5 AG.

While preferred embodiments of the present invention have been described, the embodiments disclosed are illustrative and not restrictive, and the invention is intended to be defined by the appended claims.

**Claims**

1. A method of calibrating a spectroscopic device for providing a non-invasive measurement of an analyte level in a test sample, comprising:

   (a) providing a plurality of calibration algorithms;
   (b) taking a set of non-invasive spectral measurements using the spectroscopic device and corresponding reference measurements of the analyte concentration for each of a plurality of samples;
   (c) using each of the calibration algorithms to calculate a set of predicted analyte concentration levels for the non-invasive spectral measurements taken in step (b);
   (d) for each set of predicted analyte concentration levels obtained in step (c) determining the following:

a range of the analyte concentration level variability;

a slope of predicted analyte concentration versus corresponding reference analyte concentration;

$R^2$ wherein R is the correlation of the predicted analyte concentration with the reference analyte concentration; and

a standard error of prediction (SEP) wherein SEP is the square root of the mean of the squared deviations of the reference analyte concentrations from the predicted analyte concentrations; and

(e) selecting a calibration algorithm suitable for calibrating the spectroscopic device;

**characterised in that** the selection step comprises:

(i) selecting the sets of predicted analyte concentration levels in which the SEP is less than an upper error limit, the variability range is greater than a lower range limit, and the slope is between a lower slope limit and an upper slope limit, the lower and upper slope limits defining an acceptable slope range;

(ii) for each of the predicted sets selected in step (i), calculating a suitability score in response to the slope, the $R^2$ and the SEP for that predicted set, and selecting the calibration algorithm corresponding to the predicted set having the optimal, i.e. the highest, suitability score as the suitable calibration algorithm;

(iii) if no predicted sets are selected in step (i), selecting the sets of predicted analyte concentration levels in which the variability range is lower than the lower range limit and in which the SEP is less than the upper error limit;

(iv) from the predicted sets selected in step (iii), selecting the calibration algorithm corresponding to the predicted set having lowest SEP as the suitable calibration algorithm; and

(v) if no predicted sets are selected in step (i) and (iii), determining that no calibration algorithm is suitable.

2. A method according to claim 1, wherein the acceptable slope range is subdivided into a plurality of subranges corresponding to a plurality of levels, comprising a first level and one or more subsequent levels, and if no predicted sets are selected in the first level, the method then includes repeating step (i) at each subsequent level until a predicted set is selected or there are no more subsequent levels.

3. A method according to claim 1, wherein the lower slope limit is less than one and the upper slope limit is greater than one.

4. A method according to claim 3, wherein the lower slope limit is about 0.3 and the upper slope limit is about 1.05.

5. A method according to claim 2, wherein the acceptable slope range is defined by a lower slope limit of about 0.3 and an upper slope limit of about 1.05.

6. A method according to any preceding claim, wherein the plurality of calibration algorithms in step (a) are generated by the steps of:

(i) compiling data sets for each of a plurality of individuals by taking non-invasive spectral measurements using the spectroscopic device and corresponding reference measurements of analyte concentration for each of a number of samples from each individual;

(ii) rejecting data sets that are not suitable for calibration, wherein the data sets not suitable for calibration comprise:

data sets that contain at least some non-invasive spectral measurements that saturate the spectroscopic device;

data sets with a small range of variability of the non-invasive spectral measurements; and/or

data sets that are correlated to another variable;

(iii) combining data sets that are suitable for calibration into a plurality of groups depending on whether correlations of the combined data sets meet predetermined criteria; and

(iv) generating a calibration algorithm for each of the groups of data sets.

7. A method according to claim 6, wherein the predetermined criteria in step (iii) include minimizing correlations of the analyte level in the combined data sets in a particular group with other parameters and maximizing the correlation between data sets in a particular group.

8. A method according to claim 6, wherein steps (iii) and (iv) are performed using partial least-squares regression analysis.

9. A method according to any preceding claim, wherein steps (a), (c), and (d) are performed on a computer associated with the spectroscopic device.

10. A method according to any preceding claim, wherein the test sample is an individual patient, the spectroscopic device is a near-infrared spectrophotometer, and the analyte is selected from the group consisting of glucose, hemoglobin, albumin, cholesterol, and ethanol.

**Patentansprüche**

1. Verfahren zum Kalibrieren einer Spektroskopievorrichtung zum Bereitstellen einer nicht-invasiven Messung eines Analyt-Niveaus bei einer Testprobe, umfassend:

(a) Bereitstellen einer Vielzahl von Kalibrierungsalgorithmen;
(b) Aufnehmen eines Satzes von nicht-invasiven Spektralmessungen unter Verwendung der Spektroskopievorrichtung und entsprechender Referenzmessungen der Analytkonzentration für jede einer Vielzahl von Proben;
(c) Verwenden jedes der Kalibrierungsalgorithmen, um einen Satz von vorhergesagten Analytkonzentrationsniveaus für die in Schritt (b) aufgenommenen nicht-invasiven Spektralmessungen zu berechnen;
(d) für jeden Satz von in Schritt (c) erhaltenen vorhergesagten Analytkonzentrationsniveaus, Ermitteln des Folgenden:

Eines Bereichs der Variabilität des Analytkonzentrationsniveaus;
Einer Steigung der vorhergesagten Analytkonzentration gegenüber der entsprechenden Referenzanalytkonzentration;
$R^2$, wobei R die Korrelation der vorhergesagten Analytkonzentration mit der Referenzanalytkonzentration ist; und
Eines Standardfehlers der Vorhersage (SEP), wobei der SEP die Quadratwurzel des Mittels der quadrierten Abweichungen der Referenzanalytkonzentrationen von den vorhergesagten Analytkonzentrationen ist; und

(e) Auswählen eines zum Kalibrieren der Spektroskopievorrichtung geeigneten Kalibrierungsalgorithmus;

**dadurch gekennzeichnet, dass** der Auswahlschritt umfasst:

(i) Auswählen der Sätze von vorhergesagten Analytkonzentrationsniveaus, bei denen der SEP kleiner als eine obere Fehlergrenze ist, der Variabilitätsbereich größer als eine untere Bereichsgrenze ist, und die Steigung zwischen einer unteren Steigungsgrenze und einer oberen Steigungsgrenze liegt, wobei die untere und die obere Steigungsgrenze einen akzeptablen Steigungsbereich definieren;
(ii) Berechnen einer Eignungsbewertung für jeden der in Schritt (i) ausgewählten vorhergesagten Sätze in Antwort auf die Steigung, das $R^2$ und den SEP für diesen vorhergesagten Satz, und Auswählen des Kalibrierungsalgorithmus, der dem vorhergesagten Satz mit der optimalen, das heißt der höchsten, Eignungsbewertung entspricht, als den geeigneten Kalibrierungsalgorithmus;
(iii) wenn keine vorhergesagten Sätze in Schritt (i) ausgewählt werden, Auswählen der Sätze von vorhergesagten Analytkonzentrationsniveaus, bei denen der Variabilitätsbereich kleiner als die untere Bereichsgrenze ist und bei denen der SEP kleiner als die obere Fehlergrenze ist;
(iv) von den in Schritt (iii) ausgewählten vorhergesagten Sätzen, Auswählen des Kalibrierungsalgorithmus, der dem vorhergesagten Satz mit dem geringsten SEP entspricht, als den geeigneten Kalibrierungsalgorithmus; und
(v) wenn keine vorhergesagten Sätze in Schritt (i) und (iii) ausgewählt werden, Feststellen, dass kein Kalibrierungsalgorithmus geeignet ist.

2. Verfahren nach Anspruch 1, bei dem der akzeptable Steigungsbereich in eine Vielzahl von Unterbereichen entsprechend einer Vielzahl von Niveaus unterteilt ist, die ein erstes Niveau und ein oder mehrere darauffolgende Niveaus umfassen, und bei dem, wenn keine vorhergesagten Sätze im ersten Niveau ausgewählt werden, das Verfahren dann das Wiederholen von Schritt (i) bei jedem darauffolgenden Niveau umfasst, bis ein vorhergesagter Satz ausgewählt wird oder es keine darauffolgenden Niveaus mehr gibt.

3. Verfahren nach Anspruch 1, bei dem die untere Steigungsgrenze kleiner als Eins ist und die obere Steigungsgrenze größer als Eins ist.

4. Verfahren nach Anspruch 3, bei dem die untere Steigungsgrenze ungefähr 0,3 ist und die obere Steigungsgrenze ungefähr 1,05 ist.

5. Verfahren nach Anspruch 2, bei dem der akzeptable Steigungsbereich durch eine untere Steigungsgrenze von ungefähr 0,3 und eine obere Steigungsgrenze von ungefähr 1,05 definiert ist.

6. Verfahren nach einem der vorangehenden Ansprüche, bei dem die Vielzahl von Kalibrierungsalgorithmen in Schritt (a) durch die folgenden Schritte erzeugt werden:

(i) Kompilieren von Datensätzen für jedes einer Vielzahl von Individuen durch Aufnehmen nicht-invasiver Spektralmessungen unter Verwendung der Spektroskopievorrichtung und entsprechender Referenzmessungen der Analytkonzentration für jede einer Anzahl von Proben von jedem Individuum;

(ii) Verwerfen von Datensätzen, die für eine Kalibrierung nicht geeignet sind, wobei die nicht für eine Kalibrierung geeigneten Datensätze umfassen:

Datensätze, die wenigstens einige nicht-invasive Spektralmessungen enthalten, welche die Spektroskopievorrichtung sättigen; Datensätze mit einem kleinen Variabilitätsbereich der nicht-invasiven Spektralmessungen; und/oder Datensätze, die mit einer anderen Variablen korreliert sind;

(iii) Kombinieren von für eine Kalibrierung geeigneten Datensätzen in einer Vielzahl von Gruppen in Abhängigkeit davon, ob Korrelationen der kombinierten Datensätze vorgegebenen Kriterien entsprechen; und

(iv) Erzeugen eines Kalibrierungsalgorithmus für jede der Datensatzgruppen.

7. Verfahren nach Anspruch 6, bei dem die vorgegebenen Kriterien in Schritt (iii) das Minimieren von Korrelationen des Analytniveaus in den kombinierten Datensätzen in einer bestimmten Gruppe mit anderen Parametern und das Maximieren der Korrelation zwischen Datensätzen in einer bestimmten Gruppe umfassen.

8. Verfahren nach Anspruch 6, bei dem die Schritte (iii) und (iv) unter Verwendung einer Regressionsanalyse der partiellen kleinsten Quadrate ausgeführt werden.

9. Verfahren nach einem der vorangehenden Ansprüche, bei dem die Schritte (a), (c) und (d) auf einem mit der Spektroskopievorrichtung verbundenen Computer ausgeführt werden.

10. Verfahren nach einem der vorangehenden Ansprüche, bei dem die Testprobe ein individueller Patient ist, die Spektroskopievorrichtung ein Nahinfrarot-Spektrophotometer ist, und das Analyt aus der Gruppe aus Glucose, Hämoglobin, Albumin, Cholesterol und Ethanol ausgewählt ist.

## Revendications

1. Procédé d'étalonnage d'un dispositif spectroscopique pour fournir une mesure non invasive d'un niveau d'analyte dans un échantillon d'essai, comprenant

(a) la fourniture d'une pluralité d'algorithmes d'étalonnage ;

(b) la prise d'un ensemble de mesures spectrales non invasives utilisant le dispositif spectroscopique et de mesures de référence correspondantes de la concentration d'analyte pour chacun d'une pluralité d'échantillons :

(c) l'utilisation de chacun des algorithmes d'étalonnage pour calculer un ensemble de niveaux de concentration d'analyte prédits pour les mesures spectrales non invasives prises dans l'étape (b) ;

(d) pour chaque ensemble de niveaux de concentration d'analyte prédits obtenus dans l'étape (c), la détermination de ce qui suit :

une gamme de la variabilité du niveau de concentration d'analyte ; une pente de la concentration d'analyte prédite en fonction de la concentration d'analyte de référence correspondante ; $R^2$ où R est la corrélation de la concentration d'analyte prédite avec la concentration d'analyte de référence ; et une erreur standard de prédiction (SEP) dans laquelle SEP est la racine carrée de la moyenne des déviations au carré des concentrations d'analyte de référence à partir des concentrations d'analyte prédites ; et

(e) la sélection d'un algorithme d'étalonnage approprié pour l'étalonnage du dispositif spectroscopique ; **caractérisé en ce que** l'étape de sélection comprend :

(i) la sélection des ensembles des niveaux de concentration d'analyte prédits dans lesquels la SEP est inférieure à une limite d'erreur supérieure, la gamme de variabilité est supérieure à une limite de gamme inférieure et la pente se situe entre une limite de pente inférieure et une limite de pente supérieure, les limites de pente inférieure et supérieure définissant une gamme de pente acceptable ;

(ii) pour chacun des ensembles prédits choisis dans l'étape (i), le calcul d'un score approprié en réponse à la pente, du $R^2$ et de la SEP pour cet ensemble prédit et la sélection de l'algorithme d'étalonnage correspondant à l'ensemble prédit ayant le score approprié optimal, c'est-à-dire le plus élevé, comme l'algorithme d'étalonnage approprié ;

(iii) si aucun ensemble prédit n'est choisi dans l'étape (i), la sélection des ensembles des niveaux de concentration d'analyte prédits dans lesquels la gamme de variabilité est inférieure à la limite de gamme inférieure et dans lesquels la SEP est inférieure à la limite d'erreur supérieure ;

(iv) à partir des ensembles prédits choisis dans l'étape (iii), la sélection de l'algorithme d'étalonnage correspondant à l'ensemble prédit ayant la SEP la plus basse comme l'algorithme d'étalonnage approprié ; et

(v) si aucun ensemble prédit n'est choisi dans les étapes (i) et (iii), la détermination qu'aucun algorithme d'étalonnage n'est approprié.

2. Procédé selon la revendication 1, dans lequel la gamme de pente acceptable est sous-divisée en une pluralité de sous-gammes correspondant à une pluralité de niveaux, comprenant un premier niveau et un ou plusieurs niveaux subséquents, et si aucun ensemble prédit n'est choisi dans le premier niveau, le procédé comprend alors la répétition de l'étape (i) à chaque niveau subséquent jusqu'à ce qu'un ensemble prédit soit choisi ou qu'il n'y ait plus de niveaux subséquents.

3. Procédé selon la revendication 1, dans lequel la limite de pente inférieure est inférieure à un et la limite de pente supérieure est supérieure à un.

4. Procédé selon la revendication 3, dans lequel la limite de pente inférieure est d'environ 0,3 et la limite de pente supérieure est d'environ 1,05.

5. Procédé selon la revendication 2, dans lequel la gamme de pente acceptable est définie par une limite de pente inférieure d'environ 0,3 et une limite de pente supérieure d'environ 1,05.

6. Procédé selon l'une quelconque des revendications précédentes, dans lequel la pluralité d'algorithmes d'étalonnage dans l'étape (a) est générée par les étapes de :

(i) la compilation d'ensembles de données pour chacune d'une pluralité de mesures spectrales non invasives prises individuelles utilisant le dispositif spectroscopique et de mesures de référence correspondantes de la concentration d'analyte pour chacun de plusieurs échantillons pour chaque individu ;

(ii) le rejet des ensembles de données qui ne sont pas appropriés pour l'étalonnage, dans lequel les ensembles de données non appropriés pour l'étalonnage comprennent :

les ensembles de données qui comportent au moins certaines mesures spectrales non invasives qui saturent le dispositif spectroscopique ;

les ensembles de données avec une petite gamme de variabilité des mesures spectrales non invasives ; et/ou

les ensembles de données qui sont en corrélation avec une autre variable ;

(iii) la combinaison des ensembles de données qui sont appropriés pour l'étalonnage dans une pluralité d'ensembles dépendant si oui ou non les corrélations des ensembles de données combinés satisfont à des critères prédéterminés ; et

(iv) la génération d'un algorithme d'étalonnage pour chacun des groupes des ensembles de données.

7. Procédé selon la revendication 6, dans lequel les critères prédéterminés dans l'étape (iii) comprennent la minimisation des corrélations du niveau d'analyte dans les ensembles de données combinés dans un groupe particulier avec d'autres paramètres et la maximisation de la corrélation entre les ensembles de données dans un groupe particulier.

8. Procédé selon la revendication 6, dans lequel les étapes (iii) et (iv) sont réalisées en utilisant l'analyse de régression des moindres carrés partielle.

9. Procédé selon l'une quelconque des revendications précédentes, dans lequel les étapes (a), (c) et (d) sont mises en oeuvre sur un ordinateur associé au dispositif spectroscopique.

10. Procédé selon l'une quelconque des revendications précédentes, dans lequel l'échantillon d'essai est un patient, le dispositif spectroscopique est un spectrophotomètre dans le proche infrarouge et l'analyte est choisi dans le groupe comprenant le glucose, l'hémoglobine, l'albumine, le cholestérol et l'éthanol.

SEP$_{max}$ = 3.07 mmol/L

**FIGURE 1**

(Prior Art)

$$SEP_{max} = 2.76 \text{ mmol/L}$$

Clarke Error Grid

| Zone | % |
|------|-------|
| A | 52.24 |
| B | 43.37 |
| C | 1.07 |
| D | 3.22 |
| E | 0.10 |

# FIGURE 2

$$SEP_{max} = 2.28 \text{ mmol/L}$$

Clarke Error Grid

| Zone | % |
|------|-------|
| A | 54.11 |
| B | 43.31 |
| C | 0.49 |
| D | 1.96 |
| E | 0.12 |

# FIGURE 3

**FIGURE 4**

(Prior Art)

**FIGURE 5**